# FASCICULE DE BREVET EUROPEEN

(11) **EP 1 228 197 B1**
(45) Date de publication et mention de la délivrance du brevet: **04.04.2007**
(21) Numéro de dépôt: 00979719.2
(22) Date de dépôt: 10.11.2000
(51) Int. Cl.: C12N 9/00, C12N 15/52, A01N 63/00, C12N 1/20, C12R 1/00

(54) **BACTERIES SYMBIOTIQUES ET LEURS APPLICATIONS**
SYMBIOTISCHE BAKTERIEN UND IHRE VERWENDUNG
SYMBIOTIC BACTERIA AND THEIR USES

(30) Priorité: 10.11.1999 FR 9914179
(43) Date de publication de la demande: 07.08.2002
(73) Titulaire: Institut de Recherche pour le Développement ( IRD), 75480 Paris Cedex 10 (FR)
(72) Inventeur: DREYFUS, Bernard, F-34920 Le Cres (FR); GIRAUD, Eric, F-34790 Grabels (FR); BOIVIN-MASSON, Catherine, F-34820 Teyran (FR)
(74) Mandataire: Peaucelle, Chantal
(86) Numéro de dépôt international: PCT/FR2000/003154
(87) Numéro de publication internationale: WO 2001/034777

(56) Documents cités:
- WO-A-99/24613
- WO-A-99/43632
- MCDONALD IAN R ET AL: "The methanol dehydrogenase structural gene mxaF and its use as a functional gene probe for methanotrophs and methylotrophs." APPLIED AND ENVIRONMENTAL MICROBIOLOGY 1997, vol. 63, no. 8, 1997, pages 3218-3224, XP000920690 ISSN: 0099-2240
- HIRAISHI AKIRA ET AL: "Phenotype and Genetic Diversity of Chlorine-Resistant Methylobacterium Strains Isolated from Various Environments." APPLIED AND ENVIRONMENTAL MICROBIOLOGY 1995, vol. 61, no. 6, 1995, pages 2099-2107, XP000920512 ISSN: 0099-2240
- DATABASE EMBL NUCLEOTIDE AND PROTEIN SEQUENCES, 2 juillet 1993 (1993-07-02), XP002140467 HINXTON, GB
- DATABASE EMBL NUCLEOTIDE AND PROTEIN SEQUENCES, 5 novembre 1999 (1999-11-05), XP002140468 HINXTON, GB
- DATABASE EMBL NUCLEOTIDE AND PROTEIN SEQUENCES, 22 novembre 1990 (1990-11-22), XP002140469 HINXTON, GB
- TANAKA YASUHIRO ET AL: "Cloning and analysis of methanol oxidation genes in the methylotroph Hyphomicrobium methylovorum GM2." FEMS MICROBIOLOGY LETTERS 1997, vol. 154, no. 2, 1997, pages 397-401, XP000920691 ISSN: 0378-1097
- CHISTOSERDOVA LUDMILA ET AL: "Molecular and mutational analysis of a DNA region separating two methylotrophy gene clusters in Methylobacterium extorquens AM1." MICROBIOLOGY (READING) 1997, vol. 143, no. 5, 1997, pages 1729-1736, XP000920660 ISSN: 1350-0872
- MACHLIN S M ET AL: "NUCLEOTIDE SEQUENCE AND TRANSCRIPTIONAL START SITE OF THE METHYLOBACTERIUM-ORGANOPHILUM XX METHANOL DEHYDROGENASE STRUCTURAL GENE" JOURNAL OF BACTERIOLOGY 1988, vol. 170, no. 10, 1988, pages 4739-4747, XP000920648 ISSN: 0021-9193
- DE LAJUDIE PHILIPPE ET AL: "Polyphasic taxonomy of rhizobia: Emendation of the genus Sinorhizobium and description of Sinorhizobium meliloti comb. nov., Sinorhizobium saheli sp. nov., and Sinorhizobium teranga sp. nov." INTERNATIONAL JOURNAL OF SYSTEMATIC BACTERIOLOGY 1994, vol. 44, no. 4, 1994, pages 715-733, XP000920657 ISSN: 0020-7713 cité dans la demande
- GREEN P N ET AL: "THREE NEW METHYLOBACTERIUM SPECIES METHYLOBACTERIUM-RHODESIANUM NEW-SPECIES METHYLOBACTERIUM-ZATMANII NEW-SPECIES AND METHYLOBACTERIUM-FIJISAWAENSE NEW-SPECIES" INTERNATIONAL JOURNAL OF SYSTEMATIC BACTERIOLOGY 1988, vol. 38, no. 1, 1988, pages 124-127, XP000920656 ISSN: 0020-7713
- CHAN H T C ET AL: "CHARACTERISATION OF A RED FORM OF METHANOL DEHYDROGENASE FROM THE MARINE METHYLOTROPH METHYLOPHAGA MARINA" FEMS MICROBIOLOGY LETTERS,NL,AMSTERDAM, vol. 97, 1 janvier 1992 (1992-01-01), pages 293-298, XP000644548 ISSN: 0378-1097
- SAMBA RAMATOULAYE THIABA ET AL: "Diversity of rhizobia nodulating Crotalaria spp. from Senegal." SYMBIOSIS 1999, vol. 27, no. 3-4, 1999, pages 259-268, XP000920579 ISSN: 0334-5114
- SY ABDOULAYE ET AL: "Methylotrophic Methylobacterium bacteria nodulate and fix nitrogen in symbiosis with legumes." JOURNAL OF BACTERIOLOGY, vol. 183, no. 1, janvier 2001 (2001-01), pages 214-220, XP000991007 ISSN: 0021-9193

### LISTE DE SEQUENCES

<110> Institut de la Recherche pour le Développement
<120> Nouvelles bactéries symbiotiques et leurs applications
<130> 59924-1226
<140>
   <141>
<150> 9914179
   <151> 1999-11-10
<160> 3
<170> PatentIn Ver. 2.1
<210> 1
   <211> 1450
   <212> ADN
   <213> Organisme Inconnu
<220>
   <223> Origine de la séquence :Methylobacterium nodulans
<400> 1
   agatcttggc tcagagcgaa cgctggcggc aggcttaaca catgcaagtc gagcgggccc 60
   tgcggggtca gcggcagacg ggtgagtaac gcgtgggaac gtgcccttcg gttcggaaca 120
   accctgggaa actagggcta ataccggata cgtccgcaag gagaaaggct tgactgccga 180
   aggatcggcc cgcgtctgat tagctggttg gtggggtaac ggctcaccaa ggcgacgatc 240
   agtagctggt ctgagaggat gatcagccac actgggactg agacacggcc cagactccta 300
   cgggaggcag cagtggggaa tattggacaa tgggcgcaag cctgatccag ccatgccgcg 360
   tgagtgatga cggccttagg gttgtaaagc tcttttcttc gggacgataa tgacggtacc 420
   ggaggaataa gccccggcta acttcgtgcc agcagccgcg gtaatacgaa gggggctagc 480
   gttgctcgga atcactgggc gtaaagggcg cgtaggcggc tcgccaagtc gggggtgaaa 540
   gcccgtggct caaccacgga atggccttcg atactggcgg gcttgagacc ggaagaggac 600
   agcggaactg cgagtgtaga ggtgaaattc gtagatattc gcaagaacac cagtggcgaa 660
   ggcggctgtc tggtccggtt ctgacgctga ggcgcgaaag cgtggggagc aaacaggatt 720
   agataccctg gtagtccacg ctgtaaacga tgaatgctag ccgttggggt gcatgcacct 780
   cagtggcgcc gctaacgctt taagcattcc gcctggggag tacggtcgca agattaaaac 840
   tcaaaggaat tgacgggggc ccgcacaagc ggtggagcat gtggtttaat tcgaagcaac 900
   gcgcagaacc ttaccatccc ttgacatggc aggctacgtg gagagattca cggttccctt 960
   cggggacctg cacacaggtg ctgcatggct gtcgtcagct cgtgtcgtga gatgttgggt 1020
   taagtcccgc aacgagcgca accctcgccc ttagttgcca tcattgagtt gggcactcta 1080
   gggggactgc cggtgataag ccgcgaggaa ggtggggatg acgtcaagtc ctcatggccc 1140
   ttacgggatg ggctacacac gtgctacaat ggcggtgaca atgggaagcg aaggggcgac 1200
   ctggagcaaa tccccaaaag ccgtctcagt tcagattgca ctctgcaact cgagtgcatg 1260
   aaggcggaat cgctagtaat cgtggatcag cacgccacgg tgaatacgtt cccgggcctt 1320
   gtacacaccg cccgtcacac catgggagtt ggtcttaccc gacggcgctg cgccaaccgc 1380
   gagggggcag gcgaccacgg tagggtcagc gactggggtg aagtcgtaac aaggtagccg 1440
   taggggaacc                                                        1450
<210> 2
   <211> 2000
   <212> ADN
   <213> Organisme Inconnu
<220>
   <223> Origine de la séquence :Methylobacterium nodulans
<400> 2
   atcctatgaa ggtgcgcgtg cgataaattg ccaaattatc ccagcatgaa attgaaattg 60
   tcctacagac acccactgcc aggtgacgta tgaacgagag catcttgagc attaataagt 120
   gatttttaaa tcttatgaag cacatataat aaaaatcatt aggaaaatac acaaacatca 180
   cggttccaat gcttctcgat gcgtgaacag aaactgctcc gcggctgtgt gggcggggat 240
   atgcagatgg gagacgcaca tgctgggtaa gattgtggtt cgttgtcgga cgggggtgtc 300
   agtggctgcc ctggcggcgc tgctgccgct cgccggccct tcggcgctcg cgaacgacaa 360
   gctcgtcgag ctgtcgaaga gcgacggaaa ctgggtgatg cccgggaaga actacgactc 420
   cgacaactac agcaagctga agcagatcaa cgccgagaac gtcaagaacc tgaaggtgtc 480
   gtggcagttc tcgaccggac tgctgaacgg ccacgaaggc gcgccgctcg tcgtcgacgg 540
   cacgatgtac gtgcacacct cgttcccgaa caacaccttc gcgctcggcc tggacgatcc 600
   gggcaagatc ctgtggcagg acaagcccaa gcagaacccg gcggcgcgct cggtggcctg 660
   ctgcgacctc gtcaaccgcg gcctcgccta ttggccgggc gacggcaaga ccccgtccct 720
   gatcctcaag acgctgctcg acggccacgt cgtggccctc aacgcccaga cgggcgagac 780
   cgtctggaag atcgagaatt ccgatatccg ggtcggctcg acgctcacca tcgcccccta 840
   tgtggtcaag gacaaggtga tcatcggctc ctcgggcgcc gaactcggcg tgcgcggcta 900
   tctcaccgcc tacgacgtcc gcacgggcga gcagaagtgg cgcgcctacg ccacgggtcc 960
   ggattccgac ctgctgctgg ccaaggactt caacatccac aacgcccatt acggccagaa 1020
   ggggctcggc acctcgacct gggagggcga cgcctggaag atcggcggcg gcaccaactg 1080
   gggctggtac gcctatgacc ccggcaccaa cctgatctat ttcggcaccg gcaacccggc 1140
   gccctggaac gagaccatgc gtccgggcga caacaagtgg acgatgacca tcttcgcccg 1200
   cgacgtcgat accggcgagg ccaagttcgg ctaccagaag acgccgcacg acgagtggga 1260
   ttacgcgggc gtcaacgtga tgatgctctc gacccagaag gaccggagcg gcaaggagcg 1320
   caagctgctg acgcatccgg accgcaacgg catcgtctac accctcgacc gcaccaacgg 1380
   cgacctgatc tccgcccaca agatcgacga caccgtcaac gtcttcaaga ccgtcgacct 1440
   caagtcgggc ctgccggtgc gcgatccgga atacggcacc cggatggacc atctcgccaa 1500
   ggacatctgt ccctccgcga tgggctatca caaccagggc cacgattcct atgatccgga 1560
   gcgcaagctc ttctacatgg gcatcaacca catctgcatg gattgggagc ccttcatgct 1620
   gccctaccgc gccgggcagt tcttcgtcgg cgcgacgctc aacatgtatc cgggcccgaa 1680
   gggcgaccgc cagaacgccg agggcctcgg ccagatcaag gcctacgacg ccatcaccgg 1740
   caagttcaaa tgggagaaga tggagcgttt cgcggtctgg ggcggaacgc tcgccaccgc 1800
   cggcaacgtg gtcttctacg gaaccctgga cggcttcatc aaggcgcgcc actccgacac 1860
   gggtgaactg ctctggaagg cgaagctgcc ctcgggcgcg atcggctatc ccgttaccta 1920
   cacccacaag ggcacccagt acgtggcgat ctactacggc gtcggcggtt ggccgggcgt 1980
   cggcctcgtc ttcgacctgc                                              2000
<210> 3
   <211> 526
   <212> ADN
   <213> Organisme Inconnu
<220>
   <223> Origine de la séquence :Methylobacterium nodulans
<400> 3
   gctggaatcg ccgaccatgc cgaactcgcc gagttcctgc gaaaggccta caacccgagt 60
   gggacgttta atgcaaggcc attcgaaggc ggtcgaagtt gggccggcgc gagacctgag 120
   gtccgtgcaa tcggttacga ctcgcgcggc gtagcggctc atatagccgc actgcgccgc 180
   ttcatcaagg ttggggcggt cgatctactc gtcgccgaac taggattgta cgcggtacgt 240
   ccggaccttg agggactgcg aatcagtcat tcaatgctcg tgatgtatcc agccctgaag 300
   gagctcggcg ttccattcgg cttcggcacg gttcggcacg cgctgcagaa acatcttaca 360
   aggctgctcg gtaaggccgg tctagcgacc atcgtgtccg gggttcgcgt gcggtccacc 420
   ctcagagata tgcgtctcga catgccgccc acgcgggtcg aggacctact gatcctagtt 480
   ttcccgattg gaaggccaat gagcgattgg ccggctgtac gataaa                 526

## Description

L'invention a pour objet de nouvelles bactéries symbiotiques et leurs applications, notamment, pour la dépollution de l'environnement.

On sait que les bactéries symbiotiques, communément appelées *rhizobia*, jouent un rôle important dans l'environnement et l'agriculture. En formant des symbioses avec les légumineuses, elles sont en effet responsables, en grande partie, de la fixation de l'azote atmosphérique sur terre.

Ces micro-organismes sont des bactéries du sol et développent, sur la plupart de 18000 espèces de la famille des légumineuses, des organes spécialisés, appelés nodules, dans lesquels elles réduisent l'azote atmosphérique en ammoniac au bénéfice de la plante-hôte.

La formation de ces nodules résulte d'un échange de signaux symbiotiques entre la plante et les bactéries. Cet échange démarre par l'exsudation par la plante de composés inducteurs (généralement des flavonoides) des gènes de nodulation.

Les bactéries produisent alors des signaux de nodulation, les facteurs Nod, qui induisent la formation de nodules sur les racines, ou, plus rarement, sur les tiges, et permettent l'infection de la plante-hôte par les bactéries.

On notera également que la présence d'une légumineuse dans un sol permet l'enrichissement du sol en *rhizobium :* en effet, d'une part la multiplication du rhizobium est favorisée par rapport aux autres bactéries dans la rhizosphère d'une légumineuse, d'autre part, à la sénescence des nodules, les rhizobiums spécifiques de la légumineuse sont relargués dans le sol.

Trois branches de *rhizobia* ont été identifiées à ce jour, à savoir la branche des *Rhizobium* / *Mesorhizobium*/ *Sinorhizobium* / *Allorhizobium,* celle d*'Azorhizobium* et celle de *Bradyrhizobium.*

De manière surprenante, les inventeurs ont mis en évidence que les légumineuses peuvent établir une symbiose fixatrice d'azote avec des bactéries n'appartenant pas aux branches pré-citées.

L'isolement et la caractérisation de ces bactéries permettent de disposer de- souches d'intérêt et d'exploiter leurs gènes ou les caractères particuliers de ces souches pour des applications de dépollution. Il est également possible de disposer de séquences nucléotidiques qui peuvent être transférées à des bactéries pour leur conférer de telles propriétés dépolluantes.

L'invention a donc pour but de fournir de nouvelles bactéries capables de former des associations symbiotiques avec des légumineuses.

Elle vise également l'obtention et la culture de ces souches bactériennes.

L'invention vise en outre la mise à profit des propriétés de ces bactéries pour former des associations utilisables pour la dépollution du sol, de l'air et de l'eau, en permettant le maintien et le développement dans l'environnement à traiter d'une bactérie symbiotique à propriétés dépolluantes.

Les bactéries selon l'invention sont des bactéries isolées de leur environnement naturel et purifiées.

Elles sont caractérisées en ce qu'il s'agit de bactéries méthylotrophes capables de former des nodules racinaires et de fixer l'azote avec des plantes de la famille des légumineuses.

Le caractère méthylotrophe facultatif des souches de l'invention les rend capables de croître rapidement sur milieu minimum--synthétique ne contenant que du méthanol comme seule source de carbone et d'énergie. Cette croissance est similaire à celle observée avec des substrats carbonés classiques comme les pyruvates, succinates et autres.

Les souches de l'invention présentent les propriétés méthylotrophes et de nodulation de souches comprenant des séquences d'ARN16S correspondant à SEQ ID N°1.

Elles présentent plus spécialement le caractère méthylotrophe de souches comprenant des séquences de nucléotides capables de coder pour la grande sous-unité de la méthanol-déshydrogénase (MDH) dont la séquence codante de la position 260 à 2000 dans le gène mxaF correspond à SEQ ID N° 2.

L'invention vise en particulier les souches du genre *Methylobacterium.* Des souches de ce type sont des *Methylobacterium nodulans* telles qu'isolées et purifiées à partir de nodules de *Crotalaria* et, notamment, la souche *Methylobacterium nodulans* ORS2060, déposée sous le n° I 2342 le 27 octobre 1999 à la CNCM (Collection Nationale de Culture de Micro-organismes) Institut Pasteur, 28 rue du Dr Roux, Paris, France.

Les mutants des souches définies ci-dessus, et notamment de la souche ORS2060, entrent également dans le cadre de l'invention dès lors qu'ils ont les propriétés de croître sur des composés monocarbonés comme seule source de carbone et de former des nodules sur des légumineuses.

Conformément à l'invention, les souches bactériennes peuvent être isolées- à partir de nodules racinaires de légumineuses en suivant par exemple le protocole décrit dans les exemples. En variante, on a recours à des *rhizobia* dont le caractère de méthylotrophie a été modifié, par exemple par simple mutagénèse, afin de le rendre exploitable. Selon encore une autre variante, on confère par transgénèse à une bactérie le caractère de méthylotrophie et les propriétés de nodulation recherchées, dont elle est naturellement dépourvue.

La croissance rapide sur méthanol telle qu'illustrée par les exemples, les profils SDS-PAGE et/ou le séquençage permettent de vérifier que les souches produites sont bien conformes à l'invention.

Grâce à leur capacité à former des associations symbiotiques avec des légumineuses, les bactéries méthylotrophes selon l'invention sont particulièrement utiles pour des applications concernant la dépollution des compartiments sol/air/eau, et notamment en pollution automobile. Les bactéries du sol permettent ainsi de dégrader notamment des composés polluants émis par les gaz d'échappement des véhicules et, de manière générale tout composé toxique ou polluant, avec comme application, par exemple, la réhabilitation de carrières orphelines.

L'invention vise ainsi la culture de légumineuses associées aux bactéries définies ci-dessus dans des zones de pollution. On procédera ainsi avantageusement à de telles cultures le long des zones de trafic en milieu urbain ou rural.

Les cultures de légumineuses seront inoculées par les bactéries possédant des propriétés méthylotrophiques et de nodulation conformément à l'invention.

Dans ces applications à la dépollution, on utilisera avec avantage comme légumineuse des crotalaires et le lotononis en zone tropicale et méditerranéenne, et des plantes fourragères, notamment les luzernes en zone tempérée et méditerranéenne.

Les bactéries se présentent avantageusement sous forme d'inoculum. Il peut s'agir d'inoculum formé d'une suspension bactérienne destinée à être pulvérisée sur une culture de légumineuse, ou encore de billes, par exemple d'alginate, renfermant des bactéries.

Dans d'autres formes de réalisation, l'inoculum bactérien enrobe la graine de légumineuse, répandue alors en semis sur le lieu à traiter. Ces inoculums en tant que tels font également partie de l'invention.

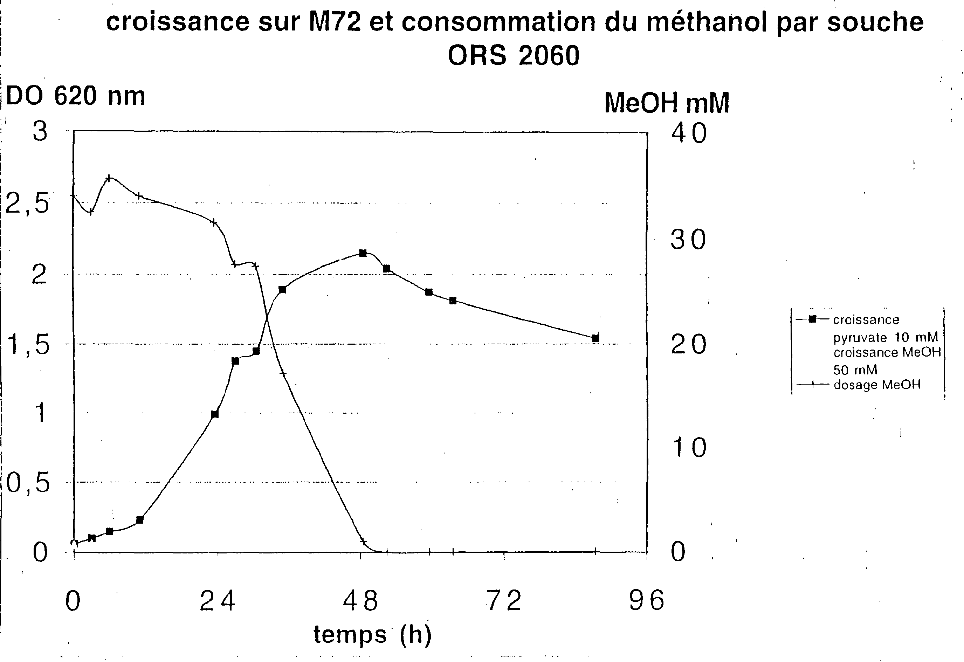

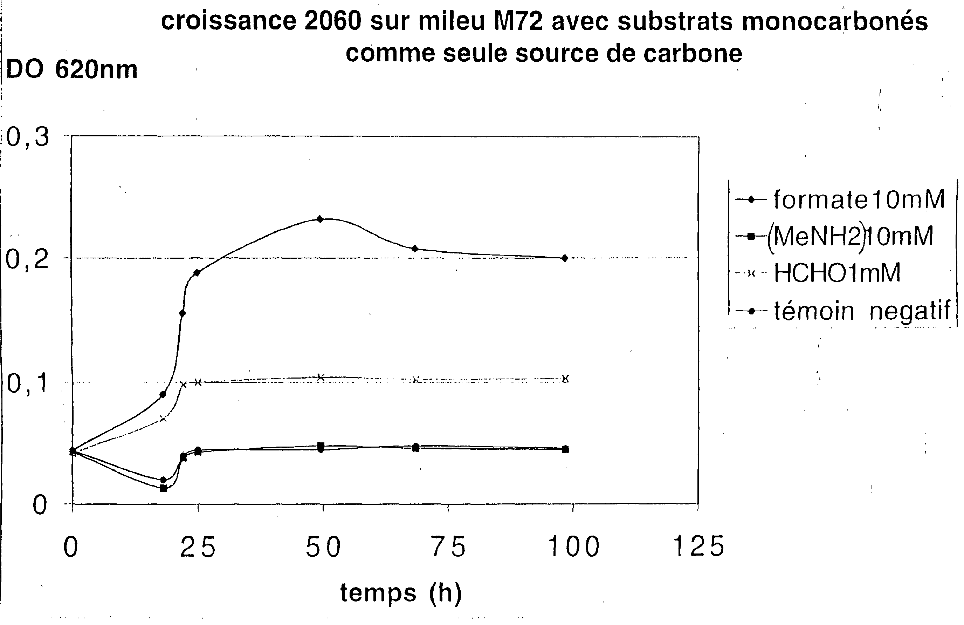

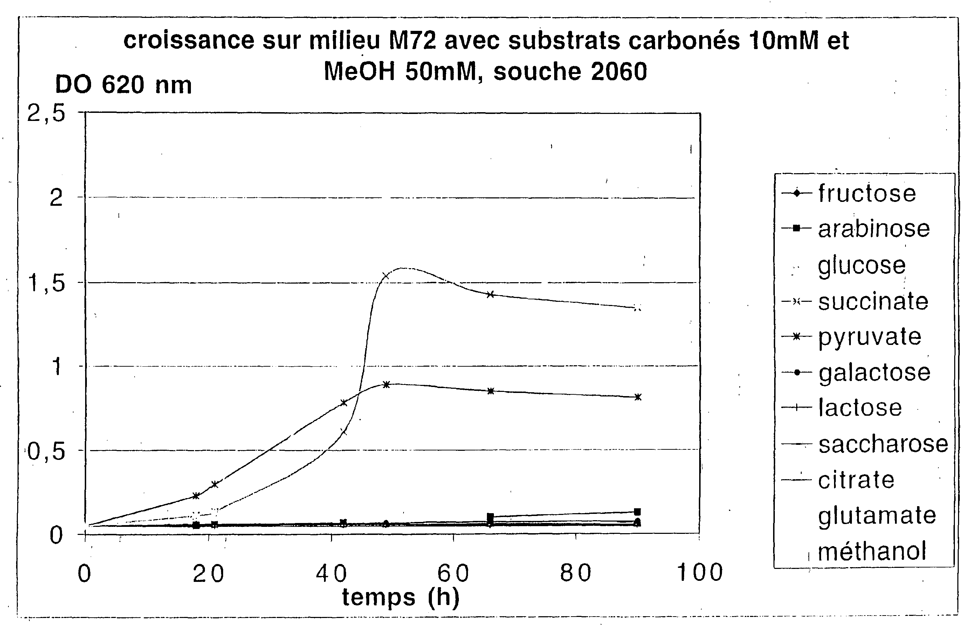

D'autres caractéristiques et avantages de l'invention seront donnés dans les exemples qui suivent, fournis à titre purement illustratif, dans lesquels il est fait référence aux figures 1 à 3 qui représentent les courbes de croissance de la souche ORS2060
- sur M 72 contenant au méthanol comme seule source de carbone (figure 1),
- sur M 72 avec des substrats monocarbonés comme seule source de carbone (figure 2),
- sur M 72 avec des substrats carbonés et du méthanol (figure 3).

### . Milieu et conditions de culture

| Milieu M 72 : | composition en g/l : |
|---|---|
| K₂HPO₄ | 1,2 g |
| CaCl₂2H₂O | 34 mg |
| MgSO₄7H2O | 0,2 g |
| Nacl | 0,1 g |
| FeCl₃6H₂O | 1 mg |
| (NH4)₂SO₄ | 0,5 g |
| oligo-éléments | 1 ml |
| Agar | 15 g |
| pH7 | |

### . Isolement de Methylobacterium nodulans ORS2060

La souche ORS2060 a été isolée d'un nodule racinaire prélevé sur un plant de *Crotalaria podocarpa* (famille des légumineuses, tribu *Crotalariae)* récolté au Sénégal. L'isolement des bactéries présentes à l'intérieur du nodule a été fait selon le protocole décrit dans l'article de Lajudie et al., 1994, Int. J. Syst. Bacteriol., 44 715-733. Les bactéries ont ensuite été purifiées par plusieurs repiquages successifs d'une colonie isolée sur milieu YM (Vincent J.M., 1970, A manual for the practical study of root nodule bacteria. International Biological Program, Handbook n° 15, p 3, Blackwell Scientific Publications, Ltd. Oxford) à 28°C. La culture pure obtenue a été inoculée sur un plant de *Crotalaria podocarpa* cultivé dans des conditions de laboratoire, puis les bactéries ont été ré-isolées d'un nodule racinaire prélevé sur le plant selon le protocole décrit. Les bactéries ont été purifiées et la culture pure obtenue (souche ORS2060) est conservée à -80°C en milieu YM additionné de glycérol 20 % final.

### . Résistance aux antibiotiques (concentration en µg/ml de milieu YMA)

| Contrôle positif : milieu YMA | culture + |
|---|---|
| Carbénicilline 100 | R |
| Ampicilline : 100 | R - |
| Néomycine 100 | S |
| Gentamycine 100 | S |
| 50 | S |
| 30 | R |
| Spectinomycine 100 - | S |
| Kanamycine 50 | S |
| Cloramphénicol 100 | S/R |
| Tétracycline 10 | S/R |
| 20 | S |
| 30 | S |
| Ac. Nalidixique 100 | R |
| Streptomycine 100 | S |

| | |
|---|---|
| Légende : R : résistance, S : sensible, S/R : non déterminé. | |

On rapporte, sur les figures 1 à 3, les courbes de croissance sur M 72 de ORS 2060.

La figure 1 donne en fonction du temps (en h) la densité optique (DO) à 620nm de la culture bactérienne et la concentration en méthanol mM.

Les résultats obtenus montrent que le méthanol est pratiquement totalement consommé après 24 h de culture et que les souches poussent sur méthanol comme seule source de carbone.

Sur la figure 2, on a rapporté les courbes de croissance (DO à 620 nm en fonction du temps en h) de la souche ORS2060 avec comme seule source de carbone du formate (10 mM), du formaldéhyde (1mM), et méthylamine (10mM).

Les résultats obtenus montrent le caractère méthylotrophe de la souche.

Sur la figure 3, on a rapporté les courbes de croissance(DO à 620 nm en fonction du temps en h), avec différents substrats carbonés à 10mM fructose, arabinose, glucose, succinate, pyruvate, glutamate, galactose, lactose, saccharose, citrate et du méthanol 50 mM.

Ces résultats confirment la forte croissance de la souche sur méthanol comparée aux autres substrats.

### . Gène de nodulation nodA d'une souche de Methylobacterium nodulans

La séquence partielle du gène nodA d'une *Methylobacterium nodulans* correspond à SEQ ID N°3.

### Utilisation d'associations de légumineuses avec des Methylobacterium nodulans comme agents anti-pollution

On procède à la culture de luzerne le long d' autoroutes dans le Sud de la France. On pulvérise sur les plants un inoculum d'une souche de *Methylobacterium nodulans* en suspension dans un milieu inerte vis-à-vis des bactéries et la luzerne. La symbiose rhizobium/légumineuse induit la formation de nodules dans lesquelles la bactérie de multiplie, puis à la sénescence des nodules une importante quantité de rhizobium est relarguée, enrichissant le sol de ces bactéries.

D'autres expérimentations comprennent la culture de Crotalaires le long des routes ou d'autoroutes.

Les mesures de pollution dans l'environnement de ces cultures, comparées à celles dans des zones avec des cultures traditionnelles comme les lauriers roses, montre l'effet bénéfique des associations suivant l'invention sur la diminution du taux de produits toxiques polluants dans l'environnement.

### . Protocole expérimental

### - Exemples de substrats testés

### Méthanol :

L'inoculum est préparé sur méthanol afin d'induire la formation de la méthanol déhydrogénase (MDH).

### Aromatiques :

Le toluène (composé important en volume de l'essence), permet de vérifier l'ouverture du cycle et l'utilisation du radical méthyle.

### Alcanes:

Comme modèle des alcanes présents comme composés de l'essence ou comme produits de la combustion des essences, on utilise des composés en C1 à C6 en mélange et/ou séparément : méthane, éthane, propane, butane, pentane et hexane en chaînes linéaires. Les 6 composés sont testés séparément. On effectue également des tests avec 2 composés séparément et ensemble : C3 et C6.

La-colonne Poraplot® est appropriée pour doser le mélange.

### Ethers :

Le méthyl t-butyl éther (MTBE) se retrouve en grande concentration dans les essences de certains pays. Parmi les méthodes de dosage disponibles, on citera celles décrites par Auria et al, 1999,Appl.Microb.Biotech., 51:498-503 et Cassada et al, Anal. Chem., 72:4654-4658.

### CO:

Le mélange test pour l'analyse des gaz d'échappement CO, CO₂ et propane par les garagistes est utilisé pour tester la dégradation du CO, ou dans d'autres essais un mélange CO/gaz inerte.Le dosage de CO et de CO₂ est effectué avec un détecteur de conductivité thermique sur la colonne Altech CTR1.

### Acétone

### Essence sans plomb (pour un test de croissance + ou -).

On peut doser l'essence par CPG (chromatographie en phase gazeuse) non discriminante produisant un seul pic exprimé en surface convertie en équivalent méthane.

Ethanol (comme référence d'un alcool).Ce test présente un intérêt pour l'utilisation des souches en biofiltres.

### Dispositif expérimental

### .Matériel

Les expériences sont réalisées en milieu liquide agité dans des flacons de 250 ml à diverticule permettant une mesure directe de la DO sans ouverture du flacon.

Les flacons sont fermés avec un dispositif en Téflon® (Mininert) permettant les injections et les prélèvements, et évitant l'adsorption des composés organiques volatiles (COV) sur le caoutchouc des bouchons classiques.

### . Traitements

On procède à une inoculation au 10ème jour avec une culture de *Methylobacterium* nodulans en fin de phase exponentielle sur milieu contenant du méthanol et de l'extrait de levure comme source de vitamines et substances de croissance. Le méthanol résiduel dans l'inoculum est dosé par CPG.

La culture est répétée 3 fois pour s'assurer que toute trace de source de carbone initiale (méthanol) a bien disparu.

Les expériences sont effectuées avec 2 répétitions :
2 contrôles sur méthanol + extrait de levure
2 contrôles sans addition de substrat
2 flacons par substrat.

Après la première culture, le meilleur des deux flacons est utilisé pour l'inoculation de deux autres flacons. Après cette seconde culture, on effectue une troisième culture en utilisant de nouveau le meilleur des deux flacons comme source d'inoculum. On effectue des contrôles également avec chaque substrat, mais sans *Methylobacterium,* pour tester d'éventuels processus de dégradation ou d'immobilisation abiotiques.

### .Mesures

Suivi de la croissance : DO à 540 nm.

En fonction de la croissance :
- suivi du COV dans l'espace de tête d'une chromatographie en phase gazeuse + Détecteur de ionisation de flamme FID (un prélèvement/jour),
- éventuellement, suivi du CO₂ et O₂ dans le cas d'une baisse du COV dans l'espace de tête,
- mesure du pH final.

### . Substrats et quantités à utiliser :

Volume du flacon : 250 ml ; Volume du diverticule : 18 ml ; Volume de la culture : 30 ml ; Volume gazeux : 220 ml soit 44 ml d'oxygène.

| **Traitement** | **Formule** | **PM** | **densité** | **réaction** | **Volmax pour 44 ml O2** |
|---|---|---|---|---|---|
| Méthanol | CH₃OH | 32 | 0,79 | CH₃OH + 3/2 O₂-> CO₂ + 2H₂O | 48 µl |
| Ethanol | C₂H₅OH | 46 | 0,79 | C₂H₅OH + 3 O₂- > 2CO₂+ 3H₂O | 35, 5 µl |
| Toluène | C₇H₃ | 92 | 0, 86 | C₇H₈ + 9 O₂ -> 7 CO₂ + 4H₂O | 21 µl |
| Méthane | CH₄ | 16 | gazeux | CH₄+2 O₂->CO₂+2H₂O | 22 ml gaz |
| Ethane | C₂H₆ | 30 | gazeux | C₂H₆ + 7/2 O₂- > 2CO₂ + 3H₂O | 12 , 6 ml gaz |
| Propane | C₃H₈ | 44 | gazeux | C₃H₈ +5 O₂- > 3CO₂ + 4H₂O | 8, 8 ml gaz |
| Butane | C₄H₁₀ | 58 | gazeux | C₄H₁₀ + 13/2 O₂-> 4CO₂ + 5H₂O | 5, 9 ml gaz |
| Hexane | C₆H₁₄ | 86 | 0,66 | C₆H₁₄ + 19/2 O₂-> 6CO₂ + 7H₂O | 25 µl |
| MTBE | C₅H₁₂O | 88 | 0,74 | C₅H₁₂O + 15/2 O₂-> 5 CO₂ + 6H₂O | 29 µl |
| Acétone | C₃H₆O | 58 | 0,79 | C₃H₆O + 4 O₂-> 3 CO₂ + 3H₂O | 30 µl |
| Essence sans plomb | C₈H₁₈ | 114 | 0,69 | C₈H₁₈ + 25/2 O₂-> 8CO₂ + 9 H₂O | 27 µl |
| CO | CO | 28 | gazeux | CO+1/2 O₂ -> CO₂ | 44 ml gaz |

## Revendications

1. Bactéries symbiotiques, isolées et purifiées, **caractérisées en ce qu'**il s'agit de bactéries méthylotrophes capables de former des nodules racinaires et de fixer l'azote avec des plantes de la famille des légumineuses.

2. Bactéries symbiotiques selon la revendication 1, **caractérisées en ce qu'**elles présentent les propriétés méthylotrophes et de modulation de souches comprenant des séquences d'ARN16S correspondant à SEQ ID N° 1.

3. Bactéries symbiotiques selon la revendication 1 ou 2, **caractérisées en ce qu'**elles présentent le caractère méthylotrophe de souches comprenant des séquences de nucléotides capables de coder pour la grande sous-unité de la méthanol-déshydrogénase dont la séquence de la position 260 à 2000 dans le gène mxaF correspond à SEQ ID N° 2.

4. Bactéries symbiotiques selon l'une quelconque des revendications précédentes, **caractérisées en ce qu'**elles appartiennent au genre *Methylobacterium.*

5. Bactéries symbiotiques selon la revendication 4, **caractérisées en ce qu'**il s'agit de *Methylobacterium nodulans* telles qu'isolées et purifiées à partir de nodules de *Crotalaria*.

6. Souche de *Methylobacterium nodulans* déposée le 27 octobre 1999 à la CNCM sous le n°I 2342 et mutants de cette souche capables de croître sur des composés monocarbonés comme seule source de carbone et de former des nodules sur des légumineuses.

7. Application des bactéries symbiotiques selon l'une quelconque des revendications précédentes pour la dépollution des compartiments sol / air/ eau, notamment en pollution automobile.

8. Application selon la revendication 7, **caractérisée en ce qu'**elle comprend la culture de légumineuses comme les crotalaires et le lotononis en zone tropicale et méditerranéenne, et comme les plantes fourragères, notamment la luzerne, en zone tempérée et méditerranéenne.

9. Application selon la revendication 7 ou 8, **caractérisée en ce que** les bactéries sont utilisées sous forme d'inoculum.

10. Inoculum bactérien comprenant des bactéries selon l'une quelconque des revendications 1 à 6, **caractérisé en ce qu'**il se présente sous forme d'une suspension de bactéries, ou encore de billes ou d'un enrobage autour de la graine de légumineuse.

## Claims

1. Isolated and purified symbiotic bacteria, **characterized in that** they are methylotrophic bacteria capable of forming root nodules and fixing nitrogen in plants of the legume family.

2. Symbiotic bacteria according to claim 1, **characterized in that** they have the methylotrophic and nodulation properties of strains comprising RNA 16S sequences corresponding to SEQ ID No. 1.

3. Symbiotic bacteria according to claim 1 or 2, **characterized in that** they have the methylotrophic character of strains comprising nucleotide sequences capable of coding for the large subunit of methanol-dehydrogenase wherein the sequence from position 260 to 2000 in the mxaF gene corresponds to SEQ ID No. 2.

4. Symbiotic bacteria according to any one of the preceding claims, **characterized in that** they belong to the *Methylobacterium* genus.

5. Symbiotic bacteria according to claim 4, **characterized in that** they are *Methylobacterium nodulans* as isolated and purified using *Crotalaria* nodules.

6. A strain of *Methylobacterium nodulans* filed on 27^{th} October 1999 at the CNCM under No. I 2342 and mutants of this strain capable of growing on monocarbon compounds as the sole carbon source and forming nodules on legumes.

7. Application of the symbiotic bacteria according to any one of the preceding claims for pollution control in areas of earth/air/water, in particular vehicle pollution.

8. Application according to claim 7, **characterized in that** it comprises the cultivation of legumes such as crotalaria and lotonis in tropical and Mediterranean regions, and such as forage plants, in particular alfalfa, in temperate and Mediterranean regions.

9. Application according to claim 7 or 8, **characterized in that** the bacteria are used in the form of an inoculum.

10. A bacterial inoculum comprising bacteria according to any one of claims 1 to 6, **characterized in that** it is in the form of a suspension of bacteria, or also beads or a coating around the legume seed.

## Patentansprüche

1. Isoliert und gereinigte symbiotische Bakterien, **dadurch gekennzeichnet dass** es sich um methylotrophe Bakterien handelt, welche fähig sind Wurzelknoten zu bilden und Stickstoff mit Pflanzen der Familie der Hülsenfrüchte zu fixieren.

2. Symbiotische Bakterien gemäß Anspruch 1, **dadurch gekennzeichnet dass** sie die methylotrophen und Wurzelknöllchen Eigenschaften, einschließlich der Sequenzen ARN16S entsprechend der SEQ ID N° 1, aufweisen.

3. Symbiotische Bakterien gemäß Anspruch 1 oder 2, **dadurch gekennzeichnet dass** sie den methylotrophen Charakter der Wurzeln einschließlich Nukleotidsequenzen aufweisen, welche in der Lage sind, für die große Untereinheit der Methanol-Dehydrogenase zu kodieren, deren Sequenz der Position 260 aus 2000 in dem mxaF Gen der SEQ ID N° 2 entspricht.

4. Symbiotische Bakterien gemäß einem der vorhergehenden Ansprüche, **dadurch gekennzeichnet dass** diese der Gattung *Methylobakterien* angehören.

5. Symbiotische Bakterien gemäß Anspruch 4, **dadurch gekennzeichnet dass** es sich um *Methylobakterium nodulans* handelt, welches derart aus den Knöllchen der *Crotalaria* isoliert und gereinigt wurde.

6. *Methylobakterium nodulans* Stamm, welcher am 27. Oktober 1999 in der CNCM unter der n° I 2342 hinterlegt wurde und Mutanten dieses Stammes, welche fähig sind auf Monokohlenstoff-Zusammensetzungen als einzige Kohlenstoffquelle zu wachsen und Knöllchen auf Hülsenfrüchten zu bilden.

7. Anwendung der symbiotischen Bakterien nach einem der vorhergehenden Ansprüche zur Entgiftung von Erd-/ Wasser-/ Luftbereichen, insbesondere von Schadstoffen von Automobilen.

8. Anwendung nach Anspruch 7, **dadurch gekennzeichnet, dass** sie die Hülsenfruchtkultur wie Crotalaria und Lotononis, welche in tropischen und mediterranen Zonen vorkommt, und wie die Futterpflanzen, insbesondere die Luzerne, welche in gemäßigten und mediterranen Zonen vorkommt, umfasst.

9. Anwendung nach Anspruch 7 oder 8, **dadurch gekennzeichnet dass** die Bakterien in Form eines Inokulums verwendet werden.

10. Impfbakterien umfassend Bakterien gemäß einem der Ansprüche 1 bis 6, **dadurch gekennzeichnet dass** sie in Form einer Bakteriensuspension vorliegen, oder noch als Kügelchen oder als eine Umhüllung um den Hülsenfrüchtesamen herum.
